(19) Europäisches Patentamt

European Patent Office

Office européen des brevets

(11) Veröffentlichungsnummer: **0 640 834 A1**

(12) **EUROPÄISCHE PATENTANMELDUNG**

(21) Anmeldenummer: **94113386.0**

(22) Anmeldetag: **26.08.94**

(51) Int. Cl.⁶: **G01N 33/03**, G01N 27/06,
A47J 37/12

(30) Priorität: **27.08.93 DE 4328966**

(43) Veröffentlichungstag der Anmeldung:
**01.03.95 Patentblatt 95/09**

(84) Benannte Vertragsstaaten:
**AT BE CH DE DK ES FR GB GR IE IT LI NL PT SE**

(71) Anmelder: **WAMSLER
GROSSKUECHENTECHNIK GmbH
Landsberger Strasse 372
D-80687 München (DE)**

(72) Erfinder: **Sagredos, Angelos N.
Lenhartzstrasse 6
D-20249 Hamburg (DE)**
Erfinder: **Neumeister, Ilmar
Innsbrucker Strasse 28
D-82194 Gröbenzell (DE)**

(74) Vertreter: **Fincke, Karl Theodor, Dipl.-Phys. Dr.
et al
Patentanwälte
H. Weickmann, Dr. K. Fincke
F.A. Weickmann, B. Huber
Dr. H. Liska, Dr. J. Prechtel, Dr. B.
Böhm,
Kopernikusstrasse 9
D-81679 München (DE)**

(54) Verfahren zur Bestimmung des Verdorbenheitsgrads von Ölen oder Fetten zum Fritieren oder Braten von Lebensmitteln.

㊼ Die Erfindung betrifft ein Verfahren zur Bestimmung des Verdorbenheitsgrads von Ölen oder Fetten zum Fritieren oder Braten von Lebensmitteln, bei dem der spezifische Ohm'sche Widerstand der Öle oder Fette gemessen und das Maß des Abfalls dieses Widerstands mit zunehmender Fritier- oder Bratzeit als Kriterium für den Verdorbenheitsgrad der Öle oder Fette verwendet wird.

EP 0 640 834 A1

Die Erfindung betrifft ein Verfahren zur Bestimmung des Verdorbenheitsgrads von Ölen oder Fetten zum Fritieren oder Braten von Lebensmitteln.

Zu einer solchen Bestimmung ist es bekannt, die Dielektrizitätskonstante der erhitzten Öle oder Fette zu messen oder nach Zusatz von Reagenzien deren Farbänderung im Laufe der Zeit.

Die nach diesen Verfahren zu bestimmenden Meßwerte haben sich häufig als unzuverlässig erwiesen, bedürfen meist eines Vergleichsöls oder eines Vergleichsfetts und sind apparativ aufwendig und zeitaufwendig.

Aus der De-Zeitschrift "Deutsche Lebensmittel-Rundschau", 69. Jhrg., Heft 12, 1973, Seiten 461 bis 466, ist ein Verfahren zur Bestimmung des Verdorbenheitsgrads von Ölen und Fetten zum Fritieren oder Braten bekannt, bei dem der Gehalt an oxidierten Fettsäuren als Kriterium herangezogen wird.

Aus der DE-Zeitschrift "Deutsche Lebensmittel-Rundschau, 70. Jhrg., Heft 2, 1974, Seiten 57 bis 65, und aus der EP 05 01 682 A2 ist es bekannt, die Haltbarkeit oder die Oxidationsstabilität von Ölen oder Fetten mittels konduktometrischer Meßzellen zu bestimmen.

Aufgabe der Erfindung ist es, ein Verfahren eingangs genannter Art anzugeben, das zu zuverlässigen Meßwerten führt, keiner Vergleichsöle oder Vergleichsfette bedarf und apparativ ohne großen Aufwand, mit verhältnismäßig geringem Zeitaufwand durchzuführen ist.

Zur Lösung dieser Aufgabe ist das Verfahren dadurch gekennzeichnet, daß der spezifische Ohmsche Widerstand der Öle oder Fette gemessen und das Maß des Abfalls dieses Widerstands mit zunehmender Fritier- oder Bratzeit als Kriterium für den Verdorbenheitsgrad der Öle oder Fette vewendet wird.

Keines Vergleichsöls oder Vergleichsfetts bedarf es, wenn als Kriterium die Differenz der Widerstände zwischen einem erstmaligen Beginn des Fritierens oder Bratens und dem jeweiligen Zeitpunkt nach dem erstmaligen Fritieren oder Braten verwendet wird oder wenn als Kriterium die Differenz der Widerstände zwischen einem vorgegebenen Zeitpunkt - vorzugsweise 4 bis 16 Stunden - nach einem erstmaligen Fritieren oder Braten und dem jeweiligen Zeitpunkt nach dem erstgenannten Zeitpunkt verwendet wird.

Der genannte vorgegebene Zeitpunkt ist abhängig von der Art des jeweiligen Öls oder Fetts und kann, wenn er einmal entsprechend ermittelt worden ist, so festgesetzt werden, daß nach diesem Zeitpunkt der Abfall der Widerstandsdifferenz linear ist oder eine lineare Interpolation gestattet, was bei einer Darstellung in einem Diagramm etwa auf einem Bildschirm dem Benutzer eine Extrapolation auf einen Zeitpunkt gestattet, bei dem das Öl oder Fett verdorben ist oder jedenfalls - nach den lebensmittelrechtlichen Bestimmungen - nicht mehr benutzt werden darf.

Selbstverständlich ist die Messung des spezifischen Ohmschen Widerstands der Messung der spezifischen Leitfähigkeit äquivalent und auch der Messung des Widerstands bzw. der Leitfähigkeit zwischen den Elektroden des jeweils verwendeten Meßgeräts.

**Messung des Verdorbenheitsgrades eines Fritierfettes oder Bratfettes mit dem spezifischen Widerstand bzw. der Leitfähigkeit**

**Beispiel 1**

Der spezifische Widerstand R ist der ohmsche Widerstand einer Flüssigkeit in einer Meßzelle, die durch die Zellkonstante c als Quotient von Elektrodenabstand d (cm) zu Elektrodenfläche F ($cm^2$)

$$c = \frac{d}{F} \quad (cm^{-1})$$

charakterisiert ist.

Aus dem Wert für R und der Zellkonstante c kann die Leitfähigkeit L des Fritierfettes durch die Formel

$$L = \frac{c}{R} \quad (Ohm^{-1} \cdot cm^{-1})$$

berechnet werden.

Die elektrische Leitfähigkeit von Fetten und Ölen ist sehr niedrig. Sie liegt im Bereich von ca. L = $10^{-12}$ Ohm$^{-1}$ • cm$^{-1}$. Aus diesem Grund wurde für die Messungen des spezifischen Widerstandes ein Gerät eingesetzt, das Widerstände bis 2 • $10^{14}$ Ohm anzeigen kann.

Dem eingesetzten Hochohmmeßgerät wurden eine Meßzelle für Flüssigkeiten mit einer Zellkonstante c = 0.114 cm$^{-1}$ und zwei flache Platinelektroden von ca. 2 cm$^2$ Fläche bei einem Abstand von ca. 0,2 cm. Die Meßspannung wurde auf 100 V Gleichspannung eingestellt. Das zu prüfende Fritierfett wurde in einem Probenglas von 20 mm-Durchmesser in einem Heizblock auf eine konstante Temperatur von 75 °C erwärmt. Von drei Einzelproben je Muster wurde der Widerstand R in Ohm ca. 1 min. nach Anlegen der Spannung gemessen und dann der Mittelwert errechnet. Probenglas und Meßzelle wurden vor jeder Messung mit einem Lösungsmittel wie z.B. Petrolether gereinigt.

Bei diesen Meßbedingungen wurde der Verdorbenheitsgrad von vollraffiniertem Sojaöl beim Fritieren von Fischstäbchen gemessen. Die Fettsäuren-Zusammensetzung der wichtigsten Fettsäuren des Sojaöles war wie folgt:

| Palmitinsäure (C16:0) | = 10,9 % |
|---|---|
| Stearinsäure (C18:0) | = 3,9 % |
| Ölsäure und Ölsäure-Isomere (C18:1) | = 22,2 % |
| Linolsäure und Linolsäure-Isomere (C18:2) | = 53,6 % |

Die Fritiertemperatur betrug 175 °C.

Die Meßwerte von spezifischem Widerstand und der daraus errechneten Leitfähigkeit sowie die Meßwerte der polaren Anteile und der Säurezahl sind in der nachstehenden Tabelle zusammengestellt worden:

| Fritierzeit [h] | spezifischer Widerstand R x $10^9$ [$\Omega$] | Leitfähigkeit S x $10^{-12}$ [$\Omega^{-1}$ • cm$^{-1}$] | Verdorbenheitsgrad polare Fettanteile* [%] | Säurezahl** [SZ] |
|---|---|---|---|---|
| 0 | 14,39 | 7,92 | 3,8 | 0,16 |
| 8 | 9,39 | 12,14 | 6,1 | 0,95 |
| 16 | 7,65 | 14,90 | 8,6 | 0,97 |
| 20 | 6,17 | 18,47 | 11,7 | 1,07 |
| 24 | 5,29 | 21,55 | 16,4 | 1,12 |
| 28 | 5,12 | 22,26 | 17,3 | 1,19 |
| 30 | 4,85 | 23,50 | 17,8 | 1,17 |
| 32 | 4,36 | 26,14 | 18,4 | 1,08 |
| 34 | 3,91 | 29,15 | 19,2 | 1,27 |
| 36 | 3,67 | 31,06 | 21,0 | 1,24 |
| 38 | 3,60 | 31,66 | 21,5 | 1,88 |
| 40 | 3,46 | 32,94 | 24,2 | 1,88 |

*) bestimmt nach der DGF-Einheitsmethode C-III 3 b.
**) bestimmt nach der DGF-Einheitsmethode C-V 2.

Der spezifische Widerstand des Sojaöles wurde durch das Fritieren von Fischstäbchen herabgedrückt und es zeigte sich eine lineare Abhängigkeit zwischen den Meßwerten von ohmschen Widerstand und den polaren Anteilen, die den Verdorbenheitsgrad zusammen mit der SZ kennzeichnen. Das Fritierfett war laut polarer Anteile erst nach 40 Std. verdorben. Der ohmsche Widerstand nahm linear von 14,39 • $10^9$ $\Omega$ auf 3,46 • $10^9$ $\Omega$ ab. Da die Leitfähigkeit der Reziprok Wert des ohmschen Widerstandes ist, wird hier eine lineare Zunahme der Leitfähigkeit von 7,92 auf 32,94 • $10^{-12}$ beobachtet.

Die Korrelation vom spezifischen Widerstand und Verdorbenheitsgrad des Fritierfettes läßt sich besser beweisen, indem der Meßwert von 8 Std.-Fritierzeit bzw. 16 Std.-Fritierzeit und nicht der Meßwert von 0 Std.-Fritierzeit (= Frischfett) als Bezugswert gesetzt wird und die folgenden Meßwerte entsprechend als Relativwerte berechnet werden. Zu beiden so normierten Meßreihen der Relativwerte wurde nach dem Verfahren der kleinsten Quadrate (Gauß) eine Ausgleichsgerade berechnet (siehe nachstehende Gleichung), gekennzeichnet durch die Steigung +/- Fehler , den Achsenabschnitt +/- Fehler und den Korrelations-Koeffizienten. Das gleiche Berechnungsverfahren wird angewandt, nachdem von den Meßwerten der natürliche Logarithmus (Basis e) berechnet wurde.

Ausgleichsgerade

$$y = m \cdot x + b$$

y = Normierter Wert auf der Ausgleichsgerade [%]
m = Steigung der Geraden
x = Fritierzeit [Stdn.]
b = Achsenabschnitt der Geraden

Die so errechneten Relativwerte sowie die normierten Werte aus der linearen bzw. der logarithmischen Regression der Meßwerte in Ohm aus dem Fritieren von Fischstäbchen in Sojaöl sind in der nachstehenden Tabelle 1 und 2 zusammengestellt worden:

| Fritierzeit (X) | spezifischer Widerstand $R \times 10^9$ | Relativwerte und Ausgleichsgerade [%] | | | | | |
|---|---|---|---|---|---|---|---|
| | | 8 Stdn. Fritierzeit | | | 16 Stdn. Fritierzeit | | |
| [Stdn.] | [Ω] | log. normiert | linear normiert | Y linear | log. normiert | linear normiert | Y linear |
| 0 | 14,39 | | | | | | |
| 8 | 9,39 | 100,00 | 100,00 | 92,8 | - | - | |
| 16 | 7,65 | 90,85 | 81,47 | 77,6 | 100,00 | 100,00 | 91,4 |
| 20 | 6,17 | 81,25 | 65,71 | 70,0 | 89,43 | 80,65 | 83,0 |
| 24 | 5,29 | 74,38 | 56,34 | 62,4 | 81,87 | 69,15 | 74,6 |
| 28 | 5,12 | 72,92 | 54,53 | 54,8 | 80,26 | 66,93 | 66,2 |
| 30 | 4,85 | 70,50 | 51,65 | 51,0 | 77,60 | 63,40 | 62,0 |
| 32 | 4,36 | 65,75 | 46,43 | 47,2 | 72,37 | 56,99 | 57,8 |
| 34 | 3,91 | 60,88 | 41,64 | 43,4 | 67,01 | 51,11 | 53,6 |
| 36 | 3,67 | 58,05 | 39,08 | 39,6 | 63,90 | 47,97 | 49,4 |
| 38 | 3,68 | 58,17 | 39,19 | 35,8 | 64,03 | 48,10 | 45,2 |
| 40 | 3,46 | 55,42 | 36,85 | 32,0 | 61,00 | 45,23 | 41,0 |
| Steigung (m): | | -1,4 ± 0,05 | -1,9 ± 0,12 | | -1,5 ± 0,08 | -2,1 ± 0,14 | |
| Achsenabschnitt (b): | | 111 ± 1,6 | 108 ± 3,5 | | 122 ± 2,4 | 125 ± 4,4 | |
| Korrelationskoeffizient (r): | | -0,9912 | -0,9780 | | -0,9862 | -0,9760 | |

Die Korrelationskoeffizienten bestätigen eindeutig die lineare Abnahme des spezifischen Widerstandes mit fortschreitender Fritierzeit und der damit verbundenen Qualitätsabnahme des Sojaöles.

**Beispiel 2**

Kabeljaufilet wurde mit Sojaöl folgender Fettsäurezusammensetzung

| | |
|---|---|
| Palmitinsäure (C16:0) | = 8,1 % |
| Stearinsäure (C18:0) | = 4,9 % |
| Ölsäure und Ölsäure-Isomere (C18:1) | = 27,2 % |
| Linolsäure und Linolsäure-Isomere (C18:2) | = 53,3 % |

bei 175 °C fritiert.

Der Verdorbenheitsgrad dieses Fritierfettes wurde, wie unter Beispiel 1 mittels spezifischem Widerstand gemessen.

Die Meßwerte von dem spezifischen Widerstand und die daraus errechneten Meßwerte der Leitfähigkeit sowie die Relativwerte, die auf die Meßwerte des Fritierfettes nach 8 Stdn. und 16 Stdn. Fritierzeit bezogen und normiert wurden, sind in der nachstehenden Tabelle zusammengestellt worden. Die Vergleichswerte von polaren Anteilen und SZ sind ebenfalls in der Tabelle eingetragen.

| Fritier-zeit (X) [Stdn.] | spezifischer Widerstand $R \times 10^9$ [$\Omega$] | Leitfähig-keit $5 \times 10^{-12}$ [$\Omega^{-1} \times cm^{-1}$] | Polare Anteile [%] | Säure-zahl [SZ] | Relativwerte [%] | | | |
|---|---|---|---|---|---|---|---|---|
| | | | | | 8 Stdn. | | 16 Stdn. | |
| | | | | | linear | log. | linear | log. |
| 0 | 14,39 | 7,92 | 3,8 | 0,05 | - | - | - | - |
| 8 | 8,56 | 13,31 | 7,9 | 0,32 | 100 | 100 | - | - |
| 16 | 5,85 | 19,48 | 13,1 | 0,86 | 68,34 | 82,27 | 100 | 100 |
| 20 | 4,51 | 25,27 | 13,4 | 1,10 | 52,69 | 70,15 | 77,09 | 85,27 |
| 24 | 3,68 | 30,97 | 15,2 | 1,08 | 42,99 | 60,68 | 64,91 | 73,76 |
| 28 | 3,39 | 33,62 | 16,4 | 1,61 | 39,60 | 56,86 | 57,95 | 69,11 |
| 30 | 2,89 | 39,44 | 17,1 | 1,77 | 33,76 | 49,43 | 49,40 | 60,08 |
| 32 | 2,71 | 42,06 | 18,4 | 1,81 | 31,66 | 46,43 | 46,32 | 56,44 |
| 34 | 2,61 | 43,67 | 19,4 | 1,94 | 30,49 | 44,68 | 44,62 | 54,31 |
| 36 | 2,23 | 51,12 | 20,9 | 2,07 | 26,05 | 37,35 | 38,12 | 45,40 |
| 38 | 2,10 | 54,28 | 20,2 | 2,09 | 24,53 | 34,56 | 35,90 | 42,00 |
| 40 | 2,01 | 56,71 | 25,5 | 2,61 | 23,48 | 32,52 | 34,36 | 39,52 |

| | | | |
|---|---|---|---|
| Steigung (m) | $-2,1 \pm 0,2$ | $-2,0 \pm 0,06$ | $-2,5 \pm 0,1$ | $-2,4 \pm 0,08$ |
| Achsenabschnitt (b) | $102 \pm 5,8$ | $113 \pm 1,8$ | $129 \pm 5,6$ | $135 \pm 2,5$ |
| Korrelationskoeffizient (r) | 0,9896 | -0,9949 | 0,9850 | -0,9939 |

Es ist ersichtlich, daß die Meßwerte vom spezifischen Widerstand bzw. der Leitfähigkeit in Korrelation zu dem Verdorbenheitsgrad des Fritierfettes Sojaöl, der durch die Zunahme der polaren Anteile und der SZ dokumentiert wird, stehen. Dieses wird auch durch die Ausgleichsgerade und den Korrelationskoeffezienten bestätigt.

Die Ausgleichsgerade errechnet sich nach der Gleichung (s. Beispiel 1)

$$y = m \cdot x + b$$

**Beispiel 3**

Paniertes Schweineschnitzel wurde mit einem Fritierfett folgender Fettsäurezusammensetzung

| | |
|---|---|
| Palmitinsäure (C16:0) | = 44,4 % |
| Stearinsäure (C18:0) | = 4,8 % |
| Ölsäure und Ölsäure-Isomere (C18:1) | = 36,4 % |
| Linolsäure und Linolsäure-Isomere (C18:2) | = 10,3 % |

bei 175 °C fritiert.

Der Verdorbenheitsgrad dieses Fritierfettes wurde, wie unter Beispiel 1 mittels spezifischem Widerstand gemessen.

Die Meßwerte von dem spezifischen Widerstand und die daraus errechneten Meßwerte der Leitfähigkeit sowie die Relativwerte, die auf die Meßwerte des Fritierfettes nach 8 Stunden und 16 Stunden Fritierzeit bezogen und normiert wurden, sind in der nachstehenden Tabelle zusammengestellt worden:

| Fritier-zeit (X) [Stdn.] | spezifischer Widerstand R x 10$^9$ [$\Omega$] | Leitfähig-keit 5 x 10$^{-12}$ [$\Omega^{-1}$ x cm$^{-1}$] | Polare Anteile [%] | Säure-zahl [SZ] | Relativwerte [%] | | | |
|---|---|---|---|---|---|---|---|---|
| | | | | | 8 Stdn. | | 16 Stdn. | |
| | | | | | linear | log. | linear | log. |
| 0 | 10,10 | 11,28 | 8,6 | 0,54 | - | - | - | - |
| 8 | 8,54 | 13,34 | 9,0 | 0,52 | 100 | 100 | - | - |
| 16 | 7,57 | 15,05 | 14,0 | 0,77 | 88,6 | 94,3 | 100 | 100 |
| 20 | 6,46 | 17,64 | 15,4 | 0,99 | 75,6 | 86,9 | 85,3 | 92,1 |
| 24 | 5,44 | 20,95 | 16,7 | 0,86 | 63,7 | 78,9 | 71,8 | 83,6 |
| 28 | 4,52 | 25,22 | 16,7 | 0,90 | 52,9 | 70,3 | 59,7 | 74,5 |
| 30 | 4,34 | 26,26 | 20,1 | 1,14 | 50,8 | 68,4 | 57,3 | 72,5 |
| 32 | 4,18 | 27,27 | 21,0 | 1,58 | 48,9 | 66,6 | 55,2 | 70,6 |
| 34 | 3,70 | 30,81 | 21,3 | 1,57 | 43,3 | 61,0 | 48,8 | 64,6 |
| 36 | 3,45 | 33,04 | 22,1 | 1,61 | 40,4 | 57,7 | 45,5 | 61,1 |
| 38 | 3,42 | 33,33 | 22,3 | 1,87 | 40,0 | 57,3 | 45,1 | 60,7 |
| Steigung (m) | | | | | -2,1±0,1 | -1,5±0,07 | -2,4±0,1 | -1,7±0,07 |
| Achsenabschnitt (b) | | | | | 117±2,8 | 115±1,9 | 131±4,4 | 126±2,1 |
| Korrelationskoeffizient (r) | | | | | -0,9885 | -0,9886 | -0,9814 | -0,9918 |

Es ist ersichtlich, daß die Meßwerte vom spezifischen Widerstand bzw. Leitfähigkeit in Korrelation zu dem Verdorbenheitsgrad des verwendeten Fritierfettes, der durch die Säurezahl und die polaren Anteile dokumentiert wird, stehen. Die linearen Zusammenhänge zwischen Fritierfett-Qualitätsabnahme und den Meßwerten werden durch den Korrelationskoeffizienten bestätigt.

Die Ausgleichsgerade errechnet sich nach der Gleichung (s. Beispiel 1)

$$y = m \cdot x + b$$

**Beispiel 4**

Pommes Frittes wurden mit einem Fritierfett folgender Fettsäurezusammensetzung

| | |
|---|---|
| Palmitinsäure (C16:0) | = 44,4 % |
| Stearinsäure (C18:0) | = 4,8 % |
| Ölsäure und Ölsäure-Isomere (C18:1) | = 36,4 % |
| Linolsäure und Linolsäure-Isomere (C18:2) | = 10,3 % |

bei 175 °C fritiert.

Der Verdorbenheitsgrad dieses Fritierfettes wurde, wie unter Beispiel 1 mittels spezifischem Widerstand gemessen.

Die Meßwerte von dem spezifischen Widerstand und die daraus errechneten Meßwerte der Leitfähigkeit sowie die Relativwerte, die auf die Meßwerte des Fritierfettes nach 8 Stunden und 16 Stunden Fritierzeit bezogen und normiert wurden, sind in der nachstehenden Tabelle zusammengestellt worden:

| Fritier-zeit (X) [Stdn.] | spezifischer Widerstand R x 10$^9$ [$\Omega$] | Leitfähig-keit 5 x 10$^{-12}$ [$\Omega^{-1}$ x cm$^{-1}$] | Polare Anteile [%] | Säure-zahl [SZ] | Relativwerte [%] | | | |
|---|---|---|---|---|---|---|---|---|
| | | | | | 8 Stdn. | | 16 Stdn. | |
| | | | | | linear | log. | linear | log. |
| 0 | 15,89 | 7,17 | 8,7 | 0,10 | - | - | - | - |
| 8 | 11,99 | 9,50 | 11,9 | 0,21 | 100 | 100 | - | - |
| 16 | 9,20 | 12,39 | 14,5 | 0,54 | 76,7 | 76,7 | 100 | 100 |
| 20 | 7,89 | 14,44 | 14,3 | 0,56 | 65,8 | 65,8 | 85,7 | 93,0 |
| 24 | 7,37 | 15,57 | 16,5 | 0,84 | 61,0 | 61,0 | 79,5 | 89,7 |
| 28 | 6,32 | 18,03 | 17,8 | 0,78 | 52,7 | 52,7 | 68,7 | 83,0 |
| 30 | 6,13 | 18,59 | 19,4 | 1,10 | 51,1 | 51,1 | 66,6 | 81,7 |
| 32 | 5,81 | 19,62 | 20,0 | 2,20 | 48,4 | 48,8 | 63,1 | 79,2 |
| 34 | 5,40 | 21,11 | 20,4 | 2,70 | 45,0 | 45,0 | 58,6 | 76,0 |
| 38 | 5,39 | 21,15 | 23,0 | 2,85 | 44,9 | 44,9 | 58,5 | 75,9 |
| 40 | 5,11 | 22,30 | 23,4 | 3,27 | 42,6 | 42,6 | 55,5 | 73,5 |
| Steigung (m) | | | | | -1,6$\pm$0,16 | -1,0$\pm$0,05 | -1,8$\pm$0,14 | -1,1$\pm$0,07 |
| Achsenabschnitt (b) | | | | | 103$\pm$4,0 | 105$\pm$1,6 | 123$\pm$4,2 | 115$\pm$2,1 |
| Korrelationskoeffizient (r) | | | | | -0,9619 | -0,9832 | -0,9693 | -0,9782 |

Die Ausgleichsgerade errechnet sich nach der Gleichung (s. Beispiel 1)

$$y = m \cdot x + b$$

Es ist ersichtlich, daß die Meßwerte vom spezifischen Widerstand bzw. Leitfähigkeit in Korrelation zu dem Verdorbenheitsgrad des verwendeten Fritierfettes, der durch die Säurezahl und die polaren Anteile dokumentiert wird, stehen.

Die linearen Zusammenhänge zwischen Fritierfett-Verdorbenheitsgrad und den Meßwerten werden auch durch den Korrelationskoeffizienten bestätigt.

**Beispiel 5**

Pommes Frittes wurden mit einem Fritierfett folgender Fettsäurezusammensetzung

| Palmitinsäure (C16:0) | = 6,0 % |
|---|---|
| Stearinsäure (C18:0) | = 7,3 % |
| Ölsäure und Ölsäure-Isomere (C18:1) | = 73,1 % |
| Linolsäure und Linolsäure-Isomere (C18:2) | = 6,8 % |

bei 175 °C fritiert.

Der Verdorbenheitsgrad dieses Fritierfettes wurde, wie unter Beispiel 1 mittels spezifischem Widerstand gemessen.

Die Meßwerte von dem spezifischen Widerstand und die daraus errechneten Meßwerte der Leitfähigkeit sowie die Relativwerte, die auf die Meßwerte des Fritierfettes nach 8 Stunden und 16 Stunden Fritierzeit bezogen und normiert wurden, sind in der nachstehenden Tabelle

| Fritier-zeit (X) [Stdn.] | spezifischer Widerstand $R \times 10^9$ [$\Omega$] | Leitfähig-keit $5 \times 10^{-12}$ [$\Omega^{-1} \times cm^{-1}$] | Polare Anteile [%] | Säure-zahl [SZ] | Relativwerte [%] | | | |
|---|---|---|---|---|---|---|---|---|
| | | | | | 8 Stdn. | | 16 Stdn. | |
| | | | | | linear | log. | linear | log. |
| 0 | 21,49 | 5,30 | 3,5 | 0,16 | - | - | - | - |
| 8 | 14,68 | 7,76 | 4,4 | 0,44 | 100 | 100 | - | - |
| 16 | 14,51 | 7,85 | 5,5 | 0,55 | 98,84 | 99,58 | 100 | 100 |
| 20 | 12,91 | 8,83 | 11,5 | 0,95 | 87,94 | 95,22 | 86,59 | 94,67 |
| 24 | 12,63 | 9,02 | 15,0 | 1,06 | 86,04 | 94,40 | 78,87 | 93,86 |
| 28 | 10,81 | 10,54 | 16,6 | 1,65 | 73,64 | 88,61 | 72,50 | 88,10 |
| 30 | 10,48 | 10,87 | 16,6 | 1,70 | 71,39 | 87,46 | 70,29 | 86,95 |
| 32 | 9,69 | 11,76 | 20,6 | 1,68 | 66,01 | 84,54 | 64,99 | 84,05 |
| 34 | 8,92 | 12,78 | 22,2 | 1,75 | 60,76 | 81,46 | 59,83 | 80,99 |
| 36 | 8,70 | 13,10 | 21,7 | 2,05 | 59,26 | 80,53 | 58,35 | 80,06 |
| 38 | 8,54 | 13,36 | 21,5 | 2,22 | 58,17 | 79,84 | 57,28 | 79,38 |
| 40 | 8,38 | 13,60 | 22,6 | 2,42 | 57,08 | 79,13 | 56,20 | 78,68 |
| Steigung (m) | | | | | $-1,6 \pm 0,12$ | $-0,8 \pm 0,06$ | $-1,9 \pm 0,1$ | $-0,9 \pm 0,04$ |
| Achsenabschnitt (b) | | | | | $121 \pm 3,6$ | $111 \pm 1,8$ | $127 \pm 2,9$ | $114 \pm 1,2$ |
| Korrelationskoeffizient (r) | | | | | $-0,9693$ | $-0,9662$ | $-0,9864$ | $-0,9900$ |

Die Ausgleichsgerade errechnet sich nach der Gleichung (s. Beispiel 1)

$$y = m \cdot x + b$$

Es ist ersichtlich, daß die Meßwerte vom spezifischen Widerstand bzw. Leitfähigkeit in Korrelation zu dem Verdorbenheitsgrad des verwendeten Fritierfettes, der durch die Säurezahl und die polaren Anteile dokumentiert wird, stehen.
Dieses wird auch durch den Korrelationskoeffizienten bestätigt.

## Beispiel 6

Schweinenacken wurde mit einem Fritierfett folgender Fettsäurezusammensetzung

| | |
|---|---|
| Palmitinsäure (C16:0) | = 6,8 % |
| Stearinsäure (C18:0) | = 6,7 % |
| Ölsäure und Ölsäure-Isomere (C18:1) | = 71,5 % |
| Linolsäure und Linolsäure-Isomere (C18:2) | = 6,3 % |

bei 175 °C fritiert.
Der Verdorbenheitsgrad dieses Fritierfettes wurde, wie unter Beispiel 1 mittels spezifischem Widerstand gemessen.
Die Meßwerte von dem spezifischen Widerstand und die daraus errechneten Meßwerte der Leitfähigkeit sowie die Relativwerte, die auf die Meßwerte des Fritierfettes nach 8 Stunden und 16 Stunden Fritierzeit bezogen und normiert wurden, sind in der nachstehenden Tabelle zusammengestellt worden:

| Fritier-zeit (X) [Stdn.] | spezifischer Widerstand R x $10^9$ [Ω] | Leitfähig-keit 5 x $10^{-12}$ [$Ω^{-1}$ x $cm^{-1}$] | Polare Anteile [%] | Säure-zahl [SZ] | Relativwerte [%] | | | |
|---|---|---|---|---|---|---|---|---|
| | | | | | 8 Stdn. | | 16 Stdn. | |
| | | | | | linear | log. | linear | log. |
| 0 | 11,14 | 10,23 | 3,1 | 0,16 | - | - | - | - |
| 8 | 9,13 | 12,48 | 3,9 | 0,53 | 100 | 100 | - | - |
| 16 | 7,21 | 15,81 | 6,6 | 0,72 | 78,97 | 89,32 | 100 | 100 |
| 20 | 6,77 | 16,83 | 6,7 | 0,76 | 74,15 | 86,48 | 93,90 | 96,81 |
| 24 | 5,97 | 19,09 | 9,2 | 1,63 | 65,39 | 80,79 | 82,80 | 90,45 |
| 28 | 5,83 | 19,55 | 10,4 | 1,85 | 58,27 | 75,58 | 73,79 | 89,33 |
| 30 | 5,09 | 22,39 | 12,3 | 1,83 | 55,75 | 73,58 | 70,60 | 82,37 |
| 32 | 4,80 | 23,75 | 12,8 | 1,78 | 52,57 | 70,93 | 66,57 | 79,40 |
| 34 | 4,30 | 26,51 | 12,8 | 1,78 | 47,10 | 65,95 | 59,64 | 73,84 |
| 36 | 3,80 | 30,00 | 12,4 | 1,74 | 41,67 | 60,36 | 52,70 | 64,58 |
| 38 | 3,55 | 32,11 | 13,0 | 1,76 | 38,88 | 57,29 | 49,24 | 61,65 |
| 40 | 3,38 | 33,71 | 13,4 | 1,83 | 37,02 | 55,07 | 46,88 | |
| Steigung (m) | | | | | -1,9±0,05 | -1,4±0,06 | -2,3±0,06 | -1,6±0,08 |
| Achsenabschnitt (b) | | | | | 112±1,5 | 113±1,8 | 138±1,9 | 129±2,6 |
| Korrelationskoeffizient (r) | | | | | -0,9955 | -0,9881 | -0,9958 | -0,9857 |

Die Ausgleichsgerade errechnet sich nach der Gleichung (s. Beispiel 1)

$$y = m \cdot x + b$$

Es ist ersichtlich, daß die Meßwerte vom spezifischen Widerstand bzw. Leitfähigkeit in Korrelation zu dem Verdorbenheitsgrad des verwendeten Fritierfettes, der durch die Säurezahl und die polaren Anteile dokumentiert wird, stehen.
Dieses wird auch durch den Korrelationskoeffizienten bestätigt.

**Patentansprüche**

1. Verfahren zur Bestimmung des Verdorbenheitsgrads von Ölen oder Fetten zum Fritieren oder Braten von Lebensmitteln,
dadurch gekennzeichnet,
daß der spezifische Ohmsche Widerstand der Öle oder Fette gemessen und das Maß des Abfalls dieses Widerstands mit zunehmender Fritier- oder Bratzeit als Kriterium für den Verdorbenheitsgrad der Öle oder Fette verwendet wird.

2. Verfahren nach Anspruch 1, dadurch gekennzeichnet, daß als Kriterium die Differenz der Widerstände zwischen einem erstmaligen Beginn des Fritierens oder Bratens und dem jeweiligen Zeitpunkt nach dem erstmaligen Fritieren oder Braten verwendet wird.

3. Verfahren nach Anspruch 1, dadurch gekennzeichnet, daß als Kriterium die Differenz der Widerstände zwischen einem vorgegebenen Zeitpunkt - vorzugsweise 4 bis 16 Stunden - nach einem erstmaligen Fritieren oder Braten und dem jeweiligen Zeitpunkt nach dem erstgenannten Zeitpunkt verwendet wird.

Europäisches
Patentamt

**EUROPÄISCHER RECHERCHENBERICHT**

Nummer der Anmeldung

EP 94 11 3386

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| Y | US-A-5 239 258 (KAUFFMAN) 24. August 1993<br>* das ganze Dokument *<br>--- | 1-3 | G01N33/03<br>G01N27/06<br>A47J37/12 |
| Y | PATENT ABSTRACTS OF JAPAN<br>vol. 12, no. 477 (P-800) 14. Dezember 1988<br>& JP-A-63 195 555 (SHOWA SANGYO CO LTD)<br>12. August 1988<br>* Zusammenfassung *<br>--- | 1-3 | |
| Y | US-A-5 089 780 (MEGERLE) 18. Februar 1992<br>* das ganze Dokument *<br>--- | 1-3 | |
| D,A | DEUTSCHE LEBENSMITTEL-RUNDSCHAU,<br>Bd.70, Nr.2, Februar 1974, STUTTGART<br>Seiten 57 - 65<br>H. HADORN, ET AL. 'Zur Bestimmung der<br>Oxydationsstabilität von Ölen und Fetten'<br>* das ganze Dokument *<br>--- | 1-3 | |
| A | US-A-4 372 980 (LUEBKE) 8. Februar 1983<br>* das ganze Dokument *<br>----- | 1-3 | |

**RECHERCHIERTE SACHGEBIETE (Int.Cl.6)**

G01N
A47J

Der vorliegende Recherchenbericht wurde für alle Patentansprüche erstellt

| Recherchenort | Abschlußdatum der Recherche | Prüfer |
|---|---|---|
| DEN HAAG | 3. Oktober 1994 | Bosma, R |